Europäisches Patentamt

(19) European Patent Office

Office européen des brevets

(11) Veröffentlichungsnummer: **0 048 335**
**B1**

# (12) EUROPÄISCHE PATENTSCHRIFT

(45) Veröffentlichungstag der Patentschrift:
31.08.83

(21) Anmeldenummer: 81106180.3

(22) Anmeldetag: 07.08.81

(51) Int. Cl.³: **C 07 C 51/58**, C 07 C 53/40,
C 07 C 53/42, B 01 J 31/18

(54) Verfahren zur Herstellung von Carbonsäurehalogeniden.

(30) Priorität: 18.09.80 DE 3035201

(43) Veröffentlichungstag der Anmeldung:
31.03.82 Patentblatt 82/13

(45) Bekanntmachung des Hinweises auf die Patenterteilung:
31.08.83 Patentblatt 83/35

(84) Benannte Vertragsstaaten:
AT BE CH DE FR GB IT LI NL

(56) Entgegenhaltungen:
EP-A-0 039 413
DE-A-947 469
DE-A-2 757 477
US-A-3 933 919

ULLMANNS ENCYKLOPÄDIE DER TECHNISCHEN
CHEMIE, 4. Auflage, Band 18, 1979, Verlag Chemie
Weinheim, DE. Kapitel: «Platinmetalle und -verbindun-
gen», Abschnitt 8. Verwendung, Absatz 8.1. Katalysatoren, Seiten 720–728

(73) Patentinhaber: HOECHST AKTIENGESELLSCHAFT,
Postfach 80 03 20, D-6230 Frankfurt am Main 80 (DE)

(72) Erfinder: Erpenbach, Heinz, Dr., Oberbuschweg 22,
D-5000 Köln 50 (DE)
Erfinder: Gehrmann, Klaus, Dr.,
Geschwister-Scholl-Strasse 32, D-5042 Erftstadt (DE)
Erfinder: Lork, Winfried,
Siegfried-von-Westerburg-Strasse 14, D-5042 Erftstadt
(DE)
Erfinder: Prinz, Peter, von-Geyr-Ring 104, D-5030 Hürth
(DE)

## Verfahren zur Herstellung von Carbonsäurehalogeniden

Die Erfindung betrifft ein Verfahren zur Herstellung von Carbonsäurehalogeniden durch Umsetzung von Alkylhalogeniden mit 1–6 C-Atomen oder Arylhalogeniden mit Kohlenmonoxid unter praktisch wasserfreien Bedingungen bei Temperaturen von 350 bis 575 K und Drucken von 1 bis 300 bar in Gegenwart eines Katalysatorsystems, welches mindestens eines der Edelmetalle Rhodium, Palladium, Iridium oder deren Verbindungen, Jod und/oder dessen Verbindungen, Methyltrialkylphosphoniumjodid und/oder Methyltriarylphosphoniumjodid, gegebenenfalls Trialkylphosphinoxid oder Triarylphosphinoxid und gegebenenfalls ein inertes organisches Lösemittel enthält, welches dadurch gekennzeichnet ist, dass man dem Reaktionsgemisch 0,02 bis 0,75 Mol Wasserstoff je Mol Kohlenmonoxid zufügt.

Bevorzugt arbeitet man in Gegenwart eines Katalysatorsystems, welches zusätzlich eine oder mehrere Verbindungen der unedlen Metalle Ce, Ti, Zr, Hf, Ge, Sn, Pb, V, Nb, Ta, As, Sb, Bi, Cr, Mo, W, Mn, Re, Fe, Co, Ni enthält.

Die japanischen Offenlegungsschriften 53/46 912 (1978), 53/63 307 (1978) und 53/63 308 (1978) beschreiben bereits Verfahren zur Herstellung von Acetylhalogeniden aus Alkylhalogeniden und Kohlenmonoxid in Gegenwart eines Katalysatorsystems, welches Verbindungen des Rhodiums oder Iridiums, ein Jodid, ein tertiäres Phosphin und/oder ein tertiäres Phosphinoxid in einem inerten Lösemittel enthält. Weiterhin kann das Katalysatorsystem Phospinate der Formel $(R)_2P(=O)OR$, Phosphorsäureester der Formel $(RO)_3PO$ oder Phosphorsäuretriamide der Formel $(R_2N)_3PO$ als Promotoren enthalten.

Dabei werden gemäss den Ausführungsbeispielen 1–8 der JP-OS 53/63 307 Ausbeuten an Acetylchlorid, bezogen auf eingesetztes Methylchlorid, zwischen 11,6 und 47,3 Mol-% erhalten. Die Raum-Zeit-Ausbeuten bzw. Katalysatorleistungen betragen zwischen 35 und 150 g Acetylchlorid/l Reaktionsvolumen · h bzw. zwischen 42 und 173 g Acetylchlorid/g Rhodium · h.

Die ältere europäische Anmeldung 0 039 413 beschreibt ein Verfahren zur Herstellung von Acetylchlorid durch Umsetzung von Methylchlorid mit Kohlenmonoxid unter praktisch wasserfreien Bedingungen bei Temperaturen von 350 bis 575 K und Drucken von 1 bis 300 bar in Gegenwart eines Katalysatorsystems, welches mindestens eines der Edelmetalle Rhodium, Palladium, Iridium oder deren Verbindungen, Jod und/oder dessen Verbindungen, ggf. Trialkylphosphinoxid oder Triarylphospinoxid, sowie ein inertes organisches Lösemittel enthält, welches dadurch gekennzeichnet ist, dass man in Gegenwart eines Katalysatorsystems arbeitet, welches n-Heptan als inertes Lösemittel und zusätzlich Methyltrialkylphosphoniumjodid und/oder Methyltriarylphosphoniumjodid und mindestens eine Verbindung des Chroms, Molybdäns oder Wolframs enthält.

Hierbei werden Ausbeuten an Acetylchlorid, bezogen auf eingesetztes Methylchlorid, zwischen 43 und 54% der Theorie erreicht. Die gefundenen Raum-Zeit-Ausbeuten bzw. Katalysatorleistungen bewegen sich zwischen 253 und 479 g Acetylchlorid/l Reaktionsvolumen · h bzw. zwischen 133 und 252 g Acetylchlorid/g Rhodium · h, je nach Konzentration und Reaktionszeit des Katalysatorsystems.

Obwohl die Raum-Zeit-Ausbeuten und Katalysatorleistungen gegenüber den genannten japanischen Offenlegungsschriften beträchtlich verbessert werden konnten, hat auch dieses Verfahren den Nachteil, allmählich in seiner Katalysatoraktivität abzufallen.

Die vorliegende Erfindung ermöglicht neben einer weiteren Steigerung der Ausbeute, Raum-Zeit-Ausbeute und Katalysatorleistung vor allem durch den Zusatz von Wasserstoff die Aufrechterhaltung einer völlig gleichbleibenden Aktivität der Carbonylierungskatalysatoren zur Herstellung von Carbonsäurehalogeniden. Selbst die nach mehrmaligem Einsatz in Abwesenheit von Wasserstoff in der Aktivität nachlassenden Katalysatoren zeigen nach Zusatz selbst geringer Mengen Wasserstoff zum Carobonylierungsgemisch nicht nur ihre alte Aktivität, sondern sie übertreffen sogar ihre Ausgangsleistung, welche durch die Promotierung des Katalysators mit einer oder auch mehreren Verbindungen der genannten unedlen Metalle massgeblich beeinflusst wird.

Die Edelmetalle Rhodium, Palladium, Iridium sowie die genannten unedlen Metalle werden bevorzugt in Form ihrer Chloride (z.B. $RhCl_3 \cdot 3\,H_2O$, $CrCl_3 \cdot 6\,H_2O$), Acetate, Carbonyle z.B. $[Re(CO)_5]_2$, oder als Komplexverbindungen, z.B. $Rh(CO)Cl[P(C_6H_5)_3]_2$, $Ir(CO)Cl[P(C_6H_5)_3]_2$, $RhCl[P(C_6H_5)_3]_3$, $[Rh(CO)_2Cl]_2$, $HIr(CO)[P(C_6H_5)_3]_3$, $HRh(CO)[P(C_6H_5)_3]_3$ eingesetzt.

Als Jodverbindung wird bevorzugt Methyljodid, aber auch Ethyljodid oder Jodwasserstoff eingesetzt.

Im Trialkylphosphinoxid und im Methyltrialkylphosphoniumjodid steht «alkyl» bevorzugt für methyl, ethyl, propyl, butyl. Im Triarylphosphinoxid und im Methyltriarylphosphoniumjodid steht «aryl» bevorzugt für phenyl.

Das Katalysatorsystem Edelmetall(-verbindung)/Unedelmetallverbindung/Jod(-Verbindung)/tert.Phosphinoxid/quaternäres Phosphoniumjodid/organisches Lösemittel wird bevorzugt im Mol-Verhältnis 1:(0–8):(1–100):(0–50):(1–100):(0–500) eingesetzt.

Je Mol Alkyl- bzw. Arylhalogenid wird vorzugsweise 0,0001–0,01 Mol Edelmetall(-verbindung) und 0–0,08 Mol der Verbindungen der genannten unedlen Metalle eingesetzt.

Je Mol Kohlenmonoxid werden erfindungsgemäss 0,02–0,75 Mol Wasserstoff dem Reaktionsgemisch zugesetzt. Höhere Wasserstoffgehalte erweisen sich als unzweckmässig, da bei vorgegebenem Gesamtdruck mit steigendem Wasserstoffgehalt der Partialdruck des Kohlenmonoxids abnimmt und so einen entgegengesetzten Effekt bezüglich Ausbeute und Leistung bewirkt.

Die Umsetzung gemäss der Erfindung erfolgt bevorzugt bei 20–180 bar und 150–250°C (423–523 K) unter Verwendung eines inerten Lösemittels, z.B. Hexan, Heptan, Octan, Toluol oder Xylol.

Das Verfahren der Erfindung kann sowohl in diskontinuierlicher als auch kontinuierlicher Betriebsweise durchgeführt werden.

Beispiel 1

a) 1,95 g $RhCl_3 \cdot 3\ H_2O$/, 7,91 g $CrCl_3 \cdot 6\ H_2O$, 3,4 g Triphenylphospinoxid, 60 g Methyltriphenylphosphoniumjodid, 15,8 g Methyljodid, 150,5 g Methylchlorid und 150,5 g n-Heptan werden in einen Autoklaven aus korrosionsbeständigem Edelstahl eingefüllt, zunächst mit einem Wasserstoffpartialdruck von 7,5 bar und dann mit einem CO-Partialdruck von 75 bar versehen (Gesamtdruck = 82,5 bar). Nach einer Reaktionszeit von 35 Minuten bei 453 K wird das Reaktionsprodukt dem Autoklaven entnommen und analysiert. Es wurden 131 g Acetylchlorid (56,0% der Theorie) ermittelt, woraus sich eine Raum-Zeit-Ausbeute von 561 g $CH_3COCl/l$ Reaktionsvolumen $\cdot$ h bzw. eine Katalysatorleistung von 295 g $CH_3COCl/g$ Rhodium $\cdot$ h errechnet.

b) (Vergleichsversuch)

1,95 g $RhCl_3 \cdot 3\ H_2O$, 7,91 g $CrCl_3 \cdot 6\ H_2O$, 3,4 g Triphenylphosphinoxid, 60 g Methyltriphenylphosphoniumjodid, 15,8 g Methyljodid, 150,5 g Methylchlorid und 150,5 g n-Heptan werden in einen Autoklaven aus korrosionsbeständigem Edelstahl eingefüllt und unter einen CO-Druck von 75 bar gesetzt. Nach einer Reaktionszeit von 35 Minuten bei 453 K wird im Reaktionsprodukt 86 g Acetylchlorid (36,8% der Theorie) ermittelt, woraus sich eine Raum-Zeit-Ausbeute von 369 g $CH_3COCl/l$ Reaktionsvolumen $\cdot$ h bzw. eine Katalysatorleistung von 194 g $CH_3COCl/g$ Rhodium $\cdot$ h errechnet.

Beispiel 2

1,95 g $RhCl_3 \cdot 3\ H_2O$, 3,4 g Triphenylphosphinoxid, 60 g Methyltriphenylphosphoniumjodid, 15,8 g Methyljodid, 150,5 g Methylchlorid und 150,5 g Toluol werden in einen Autoklaven eingesetzt mit einem Wasserstoffpartialdruck von 7,5 bar und dann mit einem CO-Partialdruck von 75 bar versehen. Nach einer Reaktionszeit von 35 Minuten bei 453 K wird ein Reaktionsprodukt von 115 g (49,0% der Theorie) Acetylchlorid gefunden, woraus sich eine Raum-Zeit-Ausbeute von 493 g $CH_3COCl/l$ Reaktionsvolumen $\cdot$ h bzw. eine Katalysatorleistung von 259 g $CH_3COCl/g$ Rhodium $\cdot$ h ergibt.

Beispiel 3

1,95 g $RhCl_3 \cdot 3\ H_2O$, 9,08 g $BiCl_3$, 60 g Methyltriphenylphosphoniumjodid, 15,8 g Methyljodid, 150,5 g Methylchlorid und 150,5 g n-Heptan werden in einen Autoklaven eingewogen, unter einen Wasserstoffdruck von 5 bar und dann unter einen CO-Druck von 75 bar gesetzt (Gesamtdruck= 80 bar). Nach einer Reaktionszeit von 35 Minuten bei 453 K wurden im Reaktionsprodukt 136,8 g Acetylchlorid (58,4% der Theorie) analysiert, woraus sich eine Raum-Zeit-Ausbeute von 586 g $CH_3COCl/l$ Reaktionsvolumen $\cdot$ h bzw. eine Katalysatorleistung von 309 g $CH_3COCl/g$ Rh $\cdot$ h errechnet.

Beispiel 4

1,95 g $RhCl_3 \cdot 3\ H_2O$, 4,67 g $FeCl_3$, 3,4 g Triphenylphosphinoxid, 60 g Methyltriphenylphosphoniumjodid, 15,8 g Methyljodid, 150,5 g Methylchlorid und 150,5 g n-Heptan werden in einen Autoklaven eingesetzt, dann mit einem Wasserstoffdruck von 19 bar, anschliessend mit einem CO-Druck von 75 bar versehen (Gesamtdruck = 94 bar). Nach einer Reaktionszeit von 35 Minuten bei 453 K wird das Reaktionsprodukt dem Autoklaven entnommen. Es werden 126 g Acetylchlorid (54% der Theorie) im Reaktionsprodukt bestimmt. Die Raum-Zeit-Ausbeute beträgt dabei 540 g $CH_3COCl/l$ Reaktionsvolumen $\cdot$ h, die Katalysatorleistung 284 g $CH_3COCl/g$ Rhodium $\cdot$ h.

Beispiel 5

1,31 g $PdCl_2$, 3,4 g Triphenylphosphinoxid, 60 g Methyltriphenylphosphoniumjodid, 15,8 g Methyljodid, 150,5 g Methylchlorid und 150,5 g Toluol werden in einen Autoklaven eingewogen, zunächst mit einem Wasserstoffdruck von 5 bar und dann mit einem CO-Druck von 75 bar versehen. Nach einer Reaktionszeit von 35 Minuten bei 453 K wird im Reaktionsprodukt 106 g Acetylchlorid (45,3% der Theorie) analysiert. Raum-Zeit-Ausbeute bzw. Katalysatorleistung errechnen sich zu 454 g $CH_3COCl/l$ Reaktionsvolumen $\cdot$ h bzw. zu 230 g $CH_3COCl/g$ Palladium $\cdot$ h.

Beispiel 6

1,95 g $RhCl_3 \cdot 3\ H_2O$, 8,82 g $[Re(CO)_5]_2$, 8 g Methyltributylphosphoniumjodid, 45 g Methyltriphenylphosphoniumjodid, 16 g Methyljodid, 150,5 g Methylchlorid und 150,5 g n-Hepatn werden in einen Autoklaven eingefüllt. Danach wird zunächst Wasserstoff bis zu einem Druck von 8 bar und dann CO bis zu einem Gesamtdruck von 83 bar (CO-Partialdruck = 75 bar) aufgedrückt. Nach einer Reaktionszeit von 35 Minuten bei 453 K wird im Reaktionsprodukt 147 g Acetylchlorid (62,8% der Theorie) gefunden, woraus sich eine Raum-Zeit-Ausbeute von 630 g $CH_3COCl/l$ Reaktionsvolumen $\cdot$ h bzw. eine Katalysatorleistung von 332 g $CH_3COCl/g$ Rhodium $\cdot$ h errechnet.

Beispiel 7

1,95 g $RhCl_3 \cdot 3\ H_2O$, 6,5 g $SnCl_2 \cdot 2\ H_2O$, 3,4 g Triphenylphosphinoxid, 60 g Methyltriphenylphosphoniumjodid, 15,8 g Methyljodid, 150,5 g Methylchlorid und 150,5 g n-Heptan werden in einen Autoklaven eingesetzt. Danach wird Wasserstoff mit einem Druck von 5 bar und anschliessend CO mit einem Druck von 75 bar aufgedrückt.

Nach einer Reaktionszeit von 35 Minuten bei 453 K wird im Reaktionsprodukt 110 g Acetylchlorid (47,0% der Theorie) gefunden. Raum-Zeit-Ausbeute und Katalysatorleistung betragen dabei 471 g $CH_3COCl$/l Reaktionsvolumen · h bzw. 248 g $CH_3COCl$/g Rhodium · h.

Beispiel 8

1,95 g $RhCl_3 \cdot 3 H_2O$, 10,7 g $CeCl_3 \cdot 7 H_2O$, 3,4 g Triphenylphosphinoxid, 60 g Methyltriphenylphosphoniumjodid, 15,8 g Methyljodid, 150,5 g Methylchlorid und 150,5 g n-Heptan werden in einen Autoklaven eingefüllt. Danach wird Wasserstoff mit einem Druck von 2,5 bar und dann CO mit einem Druck von 75 bar aufgedrückt (Gesamtdruck = 77,5 bar). Im Reaktionsprodukt werden nach einer Reaktionszeit von 35 Minuten bei 453 K 103 g Acetylchlorid (44,0% der Theorie) ermittelt, woraus sich eine Raum-Zeit-Ausbeute von 441 g $CH_3COCl$/l Reaktionsvolumen · h bzw. eine Katalysatorleistung von 232 g $CH_3COCl$/g Rhodium · h ergibt.

Beispiel 9

1,95 g $RhCl_3 \cdot 3 H_2O$, 5,46 g $TiCl_4$, 3,4 g Triphenylphosphinoxid, 60 g Methyltriphenylphosphoniumjodid, 15,8 g Methyljodid, 150,5 g Methylchlorid und 150,5g n-Octan werden in einen Autoklaven eingewogen, zunächst mit einem Wasserstoffdruck von 2 bar und dann mit einem CO-Druck von 75 bar versehen. Nach einer Reaktionszeit von 35 Minuten bei 453 K wird im Reaktionsprodukt 133 g Acetylchlorid analysiert. Bei einer Ausbeute von 56,7% Acetylchlorid, bezogen auf eingesetztes Methylchlorid, errechnet sich eine Raum-Zeit-Ausbeute von 570 g $CH_3COCl$/l Reaktionsvolumen · h bzw. eine Katalysatorleistung von 300 g $CH_3COCl$/g Rhodium · h.

Beispiel 10

1,95 g $RhCl_3 \cdot 3 H_2O$, 5,7 g $MnCl_2 \cdot 4 H_2O$, 60 g Methyltriphenylphosphoniumjodid, 15,8 g Methyljodid, 150,5 g Methylchlorid und 150,5 g n-Heptan werden in einen Autoklaven aus korrosionsbeständigem Edelstahl eingefüllt, danach mit einem Wasserstoffdruck von 8 bar und anschliessend mit einem CO-Druck von 75 bar versehen. Nach 35 Minuten Reaktionszeit bei 453 K wird im Reaktionsprodukt 134,3 g Acetylchlorid (57,4% der Theorie) gefunden, woraus sich eine Raum-Zeit-Ausbeute von 576 g $CH_3COCl$/l Reaktionsvolumen · h bzw. eine Katalysatorleistung von 303 g $CH_3COCl$/g Rhodium · h ergibt.

Beispiel 11

Der Katalysator aus Beispiel 1b wird nach Abtrennen des Reaktionsproduktes und erneuter Zugabe von Methyljodid, Methylchlorid und n-Heptan entsprechend den Angaben gemäss Beispiel 1b mehrfach wieder eingesetzt. Dabei fällt die Raum-Zeit-Ausbeute bzw. Katalysatorleistung nach 20maligem Einsatz von usprünglich 369 g $CH_3COCl$/l Reaktionsvolumen · h bzw. 194 g $CH_3COCl$/g Rhodium · h auf 245 g $CH_3COCl$/l Reaktionsvolumen · h bzw. 129 g $CH_3COCl$/g

Rhodium · h ab. Die Acetylchloridausbeute, bezogen auf eingesetztes Methylchlorid, sinkt dabei von ursprünglich 36,8% auf 24,5% der Theorie. Die Reaktionszeit betrug bei allen Versuchen 35 Minuten und die Reaktionstemperatur 453 K. Dieser in seiner Leistung nachlassende Katalysator wird nun wiederum mit 15,8 g Methyljodid, 150,5 g Methylchlorid und 150,5 g n-Heptan eingesetzt, wobei nach Zusatz eines Wasserstoffanteils von 8 bar noch 75 bar CO aufgedrückt werden. Nach einer Reaktionszeit von gleichfalls 35 Minuten bei 453 K werden im Reaktionsprodukt 127 g Acetylchlorid (54,4% der Theorie) gefunden, woraus sich eine Raum-Zeit-Ausbeute von 544 g $CH_3COCl$/l Reaktionsvolumen · h bzw. eine Katalysatorleistung von 286 g $CH_3COCl$/g Rhodium · h ergibt.

Beispiel 12

1,95 g $RhCl_3 \cdot 3 H_2O$, 7,91 g $CrCl_3 \cdot 6 H_2O$, 3,4 g Triphenylphosphinoxid, 60 g Methyltriphenylphosphoniumjodid, 15,8 g Methyljodid, 175 g Ethylchlorid und 150,5 g n-Heptan werden in einen Autoklaven eingefüllt, zunächst mit einem Wasserstoffdruck von 10 bar und dann mit einem CO-Druck von 75 bar versehen. Nach einer Reaktionszeit von 7 Stunden bei 463 K wird im Reaktionsprodukt 19 g Propionylchlorid (7,6% der Theorie) gefunden, woraus sich eine Raum-Zeit-Ausbeute von 7 g $CH_3CH_2COCl$/l Reaktionsvolumen · h bzw. eine Katalysatorleistung von 3,6 g $CH_3CH_2COCl$/g Rhodium · h errechnet.

Beispiel 13

1,95 g $RhCl_3 \cdot 3 H_2O$, 7,91 g $CrCl_3 \cdot 6 H_2O$, 3,4 g Triphenylphosphinoxid, 45 g Methyltributylphosphoniumjodid, 15,8 g Methyljodid und 300 g Methylbromid werden in einen Autoklaven eingewogen, dann mit einem Wasserstoffdruck von 10 bar und anschliessend mit einem CO-Druck von 75 bar versehen. Nach einer Reaktionszeit von 35 Minuten bei 453 K wird das Reaktionsprodukt dem Autoklaven entnommen und analysiert. Es werden 169,7 g Acetylbromid (43,7% der Theorie) ermittelt, woraus sich eine Raum-Zeit-Ausbeute von 727 g $CH_3COBr$/l Reaktionsvolumen · h bzw. eine Katalysatorleistung von 383 g $CH_3COBr$/g RH · h ergibt.

Beispiel 14

1,95 g $RhCl_3 \cdot 3 H_2O$, 5,7 g $MnCl_2 \cdot 4 H_2O$, 60 g Methyltributylphosphoniumjodid, 18 g Methyljodid, 150,5 g Methylchlorid und 150 g Toluol werden in einen Autoklaven eingefüllt. Anschliessend werden 50 bar Wasserstoff und 75 bar CO aufgedrückt. Nach 35 Minuten Reaktionszeit bei 453 K werden im Reaktionsprodukt 132 g Acetylchlorid (56,4% der Theorie) nachgewiesen. Dies entspricht einer Raum-Zeit-Ausbeute von 566 g $CH_3COCl$/l Reaktionsvolumen · h bzw. einer Katalysatorleistung von 297 g $CH_3COCl$/g Rh · h.

**Patentansprüche**

1. Verfahren zur Herstellung von Carbonsäurehalogeniden durch Umsetzung von Alkylhalogeniden mit 1–6 C-Atomen oder Arylhalogeniden mit Kohlenmonoxid unter praktisch wasserfreien Bedingungen bei Temperaturen von 350 bis 575 K und Drucken von 1 bis 300 bar in Gegenwart eines Katalysatorsystems, welches mindestens eines der Edelmetalle Rhodium, Palladium, Iridium oder deren Verbindungen, Jod und/oder dessen Verbindungen, Methyltrialkylphosphoniumjodid und/oder Methyltriarylphosphoniumjodid, gegebenenfalls Trialkylphosphinoxid oder Triarylphosphinoxid und gegebenenfalls ein inertes organisches Lösemittel enthält, dadurch gekennzeichnet, dass man dem Reaktionsgemisch 0,02 bis 0,75 Mol Wasserstoff je Mol Kohlenmonoxid zufügt.

2. Verfahren nach Anspruch 1, dadurch gekennzeichnet, dass man in Gegenwart eines Katalysatorsystems arbeitet, welches zusätzlich eine oder mehrere Verbindungen der unedlen Metalle Ce, Ti, Zr, Hf, Ge, Sn, Pb, V, Nb, Ta, As, Sb, Bi, Cr, Mo, W, Mn, Re, Fe, Co, Ni enthält.

3. Verfahren nach Anspruch 1 oder 2, dadurch gekennzeichnet, dass man das Katalysatorsystem Edelmetall(-verbindung)/Unedelmetallverbindung/Jod(-verbindung)/tert.Phosphinoxid/ quatern. Phosphoniumjodid/organ. Lösemittel im Molverhältnis 1:(0–8):(1–100):(0–50):(1–100):(0–500) einsetzt.

## Claims

1. Process for making carboxylic acid halides by reacting alkyl halides having from 1 to 6 carbon atoms or aryl halides with carbon monoxide under practically anhydrous conditions at temperatures of 350 to 575 K and under pressures of 1 to 300 bars in the presence of a catalyst system containing at least one of the noble metals rhodium, palladium, iridium or one of their compounds, iodine and/or its compounds, methyltrialkylphosphonium iodide and/or methyltriaryl phosphoniumiodide, and optionally trialkylphosphine oxide or triarylphosphine oxide as well as an inert organic solvent, which comprises adding 0.02 to 0.75 mol of hydrogen per mol of carbon monoxide to the reaction mixture.

2. Process as claimed in claim 1, wherein the reaction is effected in the presence of a catalyst system which additionally contains one or more compounds of the base metals Ce, Ti, Zr, Hf, Ge, Sn, Pb, V, Nb, Ta, As, Sb, Bi, Cr, Mo, W, Mn, Re, Fe, Co and Ni.

3. Process as claimed in claim 1 or 2, wherein the catalyst system noble metal (-compound)/ base metal compound/iodine(-compound)/tertiary phosphine oxide/quaternary phosphonium iodide/organic solvent is used in a molar ratio of 1:(0–8):(1–100):(0–50):(1–100):(0–500).

## Revendications

1. Procédé de préparation d'halogénures d'acides carboxyliques par réaction d'halogénures d'alkyles en $C_1$–$C_6$ ou d'halogénures d'aryles avec le monoxyde de carbone en conditions pratiquement anhydres à des températures de 350 à 575 K et sous des pressions de 1 à 300 bars en présence d'un système catalytique contenant au moins l'un des métaux nobles rhodium, palladium, iridium ou un de leurs composés, de l'iode et/ou un de ses composés, un iodure de méthyltrialkyl-phosphonium et/ou un iodure de méthyltriaryl-phosphonium, éventuellement un oxyde de trialkylphosphine ou un oxyde de triarylphosphine et, éventuellement, un solvant organique inerte, caractérisé en ce que l'on ajoute au mélange de réaction 0,02 à 0,75 mole d'hydrogène par mole de monoxyde de carbone.

2. Procédé selon la revendication 1, caractérisé en ce que l'on opère en présence d'un système catalytique contenant de plus un ou plusieurs composés des métaux communs Ce, Ti, Zr, Hf, Ge, Sn, Pb, V, Nb, Ta, As, Sb, Bi, Cr, Mo, W, Mn, Re, Fe, Co, Ni.

3. Procédé selon la revendication 1 ou 2, caractérisé en ce que l'on utilise le système catalytique (composé de) métal noble/composé de métal commun/(composé d')iode/oxyde de phosphine tertiaire/iodure de phosphonium quaternaire/solvant organique dans un rapport molaire de 1:(0–8):(1–100):(0–50):(1–100):(0–500).